# EUROPEAN PATENT APPLICATION

(11) **EP 2 181 718 A2**
(43) Date of publication of application: **05.05.2010**
(21) Application number: 09466022.2
(22) Date of filing: 29.09.2009
(51) Int. Cl.: A61L 2/07, A61L 2/24, A61L 2/26

(54) **Autoclave**

(30) Priority: 30.09.2008 CZ 200820373 U
(71) Applicant: Nybro s.r.o., 61200 Brno (CZ)
(72) Inventor: Pink Tomás, 61300 Brno (CZ)
(74) Representative: Kania, Frantisek

(57) **Abstract**

An autoclave, formed by a cylindrical chamber (1), which is closable at at least one face wall of the cylinder, wherein piping for media inlet and media outlet are connected to the cylindrical chamber (1). The cylindrical chamber (1) of the autoclave has in its lower part a double casing (2), which covers also the waste piping (18) from the cylindrical chamber (1) of the autoclave, and there is a space between two spaced walls of a double casing (2), wherein a heating medium supply leads into said space for a surface heating of the lower part of the cylindrical chamber (1) of the autoclave and of the waste piping (18) from the cylindrical chamber (1) of the autoclave. Technological elements of the autoclave and the media inlet and media outlet piping for the chamber of the autoclave are arranged above the cylindrical chamber (1) of the autoclave, and at least one face of the cylindrical chamber (1) is formed by a vertical bearing metal plate (30) which is welded to the chamber (1) of the autoclave forming one unit with it and which is provided with a door opening (32), around which, spaced from the door opening edge, a milled groove (26) is provided for a sealing between said vertical bearing metal plate (30) and the chamber door (33), by means of which the face of the autoclave is closable.

## Description

### Field of the art

The invention relates to an autoclave, formed by a cylindrical chamber, which is closable at at least one front wall of the cylinder, wherein piping for media inlet and media outlet are connected to the cylindrical chamber.

### Background of the invention

Autoclaves are pressure vessels used for sterilisation of objects, usually in steam.

Horizontal autoclaves are known, formed by a horizontal cylindrical chamber, which may be closed at one or both front walls of the cylinder, wherein piping for media inlet and media outlet are connected to the cylindrical chamber. The cylindrical walls of the known devices are usually provided with annular heating elements, which also stiffen the cylindrical chamber. A drawback of said devices is that there is a irregular thermal gradient inside the chamber which is caused by the heating elements. The irregular horizontal thermal gradient increases the regulating hysteresis of the temperature inside the chamber and reduces the thermal uniformity inside the chamber. The horizontal cylindrical chamber of the autoclave is usually located with its bottom 50 through 80 cm above the floor and the space above the floor and above the chamber, under the chamber and around the chamber is used for media distribution and for technological components, such as pumps. The known devices have got the media distribution piping outside the chamber, some of them over the chamber, some of them under the chamber and some sideways. A drawback of said arrangement is that it is difficult to insulate such piping and so thermal emission into the space around the chamber occurs. Such arrangement is difficult to clean and sanitate. When locating the valves at the piping it is to be a compromise between an easy accessibility and the requirement to locate the valve as near to the inlet of the piping into the chamber as possible. Waste piping from the chamber of the autoclave is another source of thermal emissions in the space outside the chamber and a source of cooling for part of the chamber near to the bottom so that a thermal inhomogeneity inside the chamber is caused. To avoid such problems the steam may be injected directly into the waste piping and or a direct heating may be applied but such a solution is too complicated and uneconomic. The location of the chamber 50 to 80 cm above the floor is disadvantageous as especially heavier pieces are difficult to get into the autoclave because of the height. To avoid any transmission of contaminated particles from one front wall of the autoclave onto the other or onto the technical equipment of the autoclave, the autoclave is provided with a sheet collar at its front part or parts, the sheet collar being sealed or attached or cemented to the chamber and its external edge is sealed to a wall of the building inside which the autoclave is arranged. A drawback of said arrangement consists in that the integrity of the sealed or cemented joints is most frequently impaired and then a channel for a contamination transmission is formed.

Usually known autoclaves have doors vertically sliding wherein the technical means for executing the vertical sliding of the door are arranged around a / or under said door. A drawback of said arrangement is that contaminants may drop from the items being inserted into and / or removed from the cylindrical chamber and they may get onto the technical means for executing the vertical sliding of the door which are arranged under the inlet opening. Technical means for sliding the door located under the door prevent any effective cleaning under the inlet opening of the chamber.

### Summary of the invention

Said drawbacks of the prior art devices are largely eliminated by an autoclave formed by a cylindrical chamber, which is closable at at least one face wall of the cylinder, wherein piping for media inlet and media outlet are connected to the cylindrical chamber, while the invention has following features: the cylindrical chamber of the autoclave - in its lower part - is provided with a double casing, which covers also a covered waste piping from the cylindrical chamber of the autoclave, and there is a space between two spaced walls of a double casing, wherein a heating medium supply leads into said space for a full area heating of the lower part of the cylindrical chamber of the autoclave. Technological elements of the autoclave and the media inlet and media outlet piping for the chamber of the autoclave are arranged above the cylindrical chamber of the autoclave. At least one face of the cylindrical chamber is formed by a vertical bearing metal plate which is welded to the chamber of the autoclave forming one unit with the chamber. Said vertical bearing metal plate is provided with a door opening, around which spaced from the door opening edge a milled groove is provided for a sealing between said vertical bearing metal plate and the chamber door, by means of which the face of the autoclave is closable.

It is advantageous to provided the two spaced walls of the double casing connected to each other at its edges only.

It is also favourable to make the walls of the milled groove diverging from each other towards the bottom of milled groove.

According to a preferred embodiment of the autoclave the door opening in the inlet face of the cylindrical chamber of the autoclave is formed as a part of a circle having a raised edge in its lower part for keeping the condensate at the bottom of the cylindrical chamber when the door of the chamber at the inlet face of the cylindrical chamber of the autoclave is opened.

According to another preferred embodiment of the autoclave the door opening in the inlet face of the cylindrical chamber of the autoclave has a lowered edge in its upper part, the lowered edge being arranged parallel to the raised edge of the door opening at the inlet face of the cylindrical chamber of the autoclave.

According to another preferred embodiment the control means of the autoclave are connected to the control system for a specification of the direction of use of the autoclave individually for each process and for a control of the processes taking place inside the autoclave.

According to yet another preferred embodiment the chamber door are attached to the vertical bearing metal plate by means of a mechanism for door sliding, which is attached to the vertical bearing metal plate above the level of the raised edge of the door opening.

According to another preferred embodiment the media distribution inside the cylindrical chamber of the autoclave is effected by means of media inlet and media outlet piping arranged inside the chamber of the autoclave.

### Brief description of the drawings

The invention shall now be described in more detail in connection with the attached drawings wherein Fig. 1 shows a schematic side view of the autoclave according to the invention, Fig. 2 shows a schematic front view of the autoclave according to the invention, Fig. 3 shows an axonometric view of the autoclave according to the invention, Fig. 4 is a front view of the opening in an exemplifying embodiment of the front panel of the chamber of the autoclave, Fig. 5 is a cross-sectional view of the front panel of the autoclave chamber, Fig. 6 shows a front view of the opening in another exemplifying embodiment of the front panel of the autoclave chamber, and Fig. 7 is a cross-sectional view of the another front panel of the autoclave chamber.

### Exemplifying embodiments of the invention

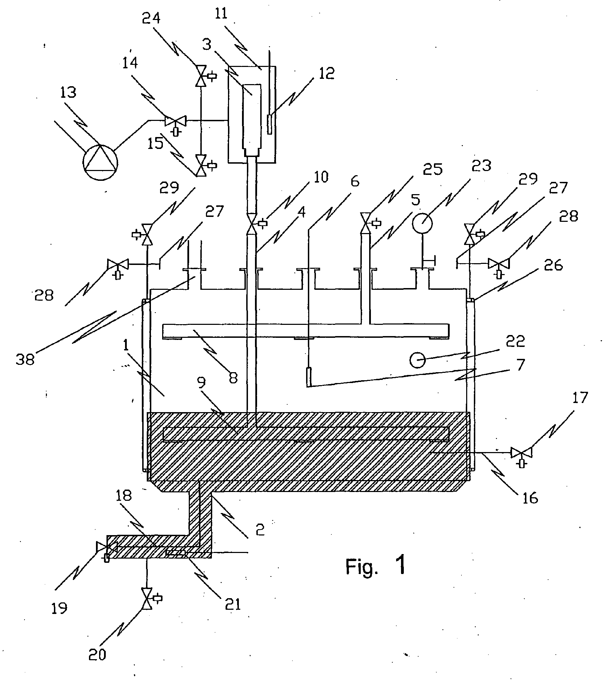

Fig. 1 shows a schematic side view of the autoclave according to the invention. A cylindrical chamber **1** is with its lower part arranged in a double casing **2**, which serves as a heating element for a uniform area heating of the lower part of the cylindrical chamber **1** of the autoclave with a constant horizontal thermal gradient. Media inlets lead into the cylindrical chamber **1** from above, wherein the media distribution is arranged inside the cylindrical chamber **1** of the autoclave. So a vacuum suction piping **4** leads via a HEPA filter **3** from above into the cylindrical chamber **1** of the autoclave. A steam injection piping **5** for steam injecting into the cylindrical chamber **1** of the autoclave and an entry **6** for a thermometer **7** lead into the cylindrical chamber **1** from above as well. A tube **9** connected to the vacuum suction piping **4** is in the lower part of the cylindrical chamber **1** and a steam distribution means **8** is in the upper part of the cylindrical chamber **1.** A shut-off valve 10 is in the vacuum section piping **4** between the cylindrical chamber **1** and the HEPA filter **3**. The HEPA filter **3** is encased in the cover **11,** inside which a thermometer **12** for the HEPA filter **3** is arranged. The vacuum suction piping **4** is connected to a vacuum pump **13** via the HEPA filter **3**. The HEPA filter **3** and the vacuum pump **13** are separated from each other by a vacuum shut-off valve **14**. A condensate outlet piping with a valve **15** for an outlet of the condensate from the cover **11** of the HEPA filter **3** is connected to the cover **11** of the HEPA filter **3.** A steam piping **16** with a steam injection valve **17** is connected to the double casing **2.** A waste piping **18** is connected to the lower part of the cylindrical chamber **1** of the autoclave and it is also arranged in the double casing **2**, in which the whole cylindrical chamber **1** is seated, wherein the waste piping **18** is provided with a waste shut-off valve **19**. A valve **20** for draining the condensate out of the double casing **2** is arranged in the double casing near to the waste piping 18 and a thermometer **21** is arranged in the double casing **2** for measuring the temperature inside the double casing **2**. A level sensor **22** is arranged inside the cylindrical chamber **1** of the autoclave. A pressure gauge **23** is arranged in the upper part of the cylindrical chamber **1** of the autoclave. A vacuum distribution means with a vacuum shut off valve **14** and a compressed air inlet with a valve **24** lead into the casing **11** of the HEPA filter **3**. The steam injection piping **5** is provided with a shut off valve **25**. The media distribution means are arranged inside the cylindrical chamber **1** of the autoclave wherein an advantage is taken of the fact that the space practically available inside the chamber is a prism, the corners of which are at the circumspection of the door opening of the cylindrical chamber **1** of the autoclave so that the distribution means arranged in the area between the wall of said imaginary prism and the wall of the cylindrical chamber **1** of the autoclave do not reduce practically the usable space inside the cylindrical chamber **1** of the autoclave. A door groove **26** is formed at a vertical bearing metal plate **30** (not shown in said Fig.) around the door opening **32** (also not shown in said Fig.) and a terminal of a pressure gauge **27** for measuring the pressure inside the door groove **26**, a vacuum inlet piping with a valve **28** and a compressed air piping with a valve **29** for compressed air lead into the door groove **26**. A safety mechanism **38** is arranged inside the upper part of the cylindrical chamber **1** of the autoclave, too, wherein it may be e.g. a safety valve or a rupture diaphragm.

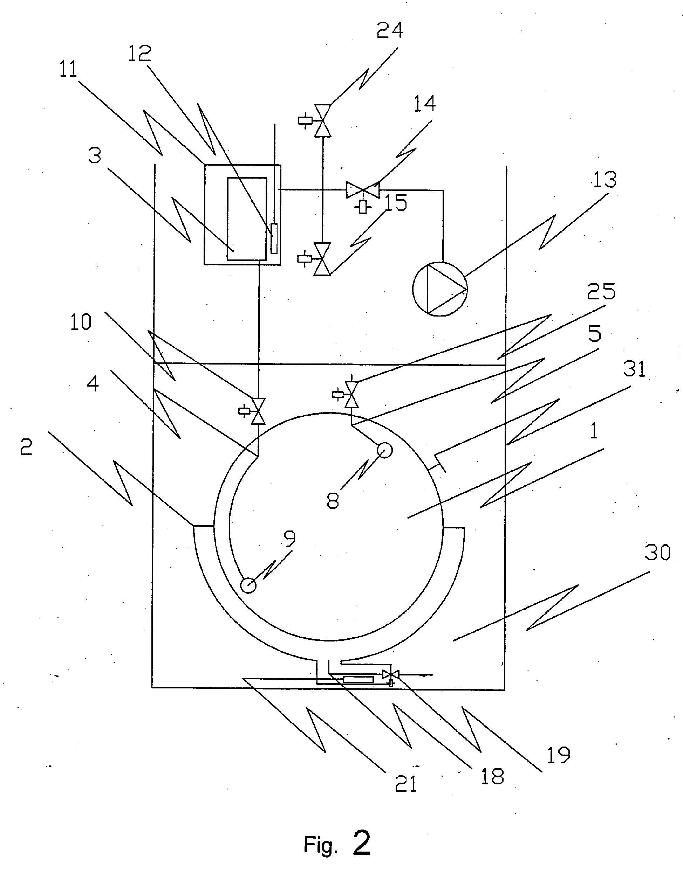

Fig. 2 shows a front view of the vertical bearing metal plate **30** which supports the cylindrical chamber **1** of the autoclave at its insertion face. The cylindrical chamber **1** of the autoclave is arranged on the vertical bearing metal plate **30** in its lower part and only a waste piping **18** with the shut-off valve for the waste from the cylindrical chamber **1** of the autoclave and with a thermometer **21** are arranged inside the double casing **2** under the vertical bearing metal plate **30**, all the other media inlet piping and control means are arranged above the cylindrical chamber **1** of the autoclave. The vacuum suction piping **4** with the tube **9** for vacuum suction in the lower part of the cylindrical chamber **1** of the autoclave lead into the cylindrical chamber **1** from above. The vacuum suction piping **4** is connected to the HEPA filter **3** via the shut-off valve **10**. Also the steam injection piping **5** leads into the cylindrical chamber **1** of the autoclave from above as well as the entry **6** for the thermometer **7** (not shown in said Fig.) for the chamber. The steam distribution means **8** is arranged in the upper part of the cylindrical chamber **1** and it is connected to the steam injection piping **5**, which is provided with a steam injection shut-off valve **25** outside the cylindrical chamber **1**. An entry **31** for the level sensor **22** leads into the cylindrical chamber **1** of the autoclave. The HEPA filter **3** and its thermometer **12** is arranged inside the cover **11** of the HEPA filter **3** above the cylindrical chamber **1** of the autoclave. The vacuum suction piping **4** is connected to the vacuum pump **13** via the HEPA filter **3**. The HEPA filter **3** and the vacuum pump **13** are separated from each other by a vacuum shut-off valve **14**. A condensate outlet piping with a valve **15** for letting the condensate out of the cover **11** of the HEPA filter **3** is connected to the HEPA filter **3**. The vacuum distribution means with a vacuum shut-off valve **14** and the compressed air feeder with a valve **24** lead into the cover **11** of the HEPA filter **3**.

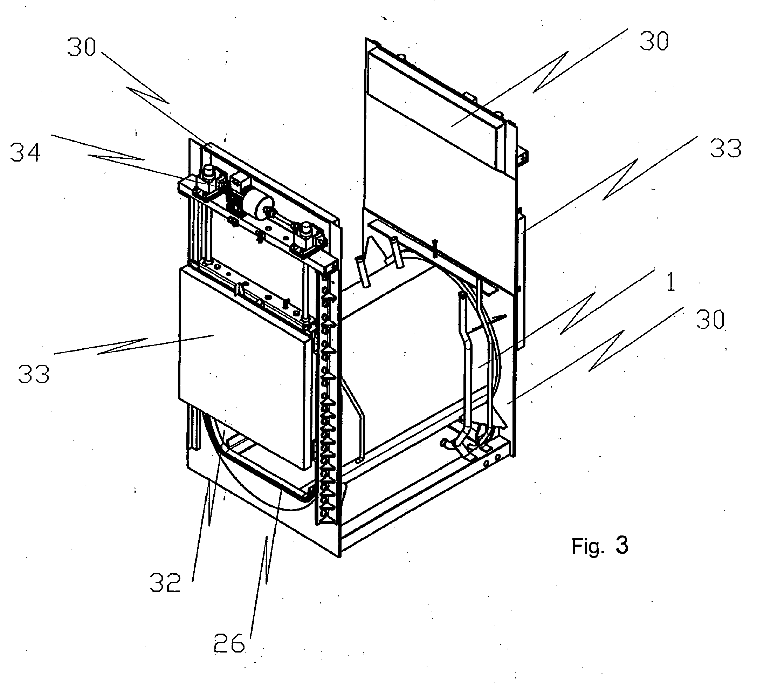

Fig. 3 shows an axonometric view of the autoclave. The faces of the cylindrical chamber **1** are welded to the vertical bearing metal plate **30**, in which the door opening **32** is formed. The door groove **26** is formed around the door opening **32** and adapted for inserting a sealing. The door opening is partially covered by the door **33**, which may be shifted in the vertical direction using a door shifting mechanism **34**.

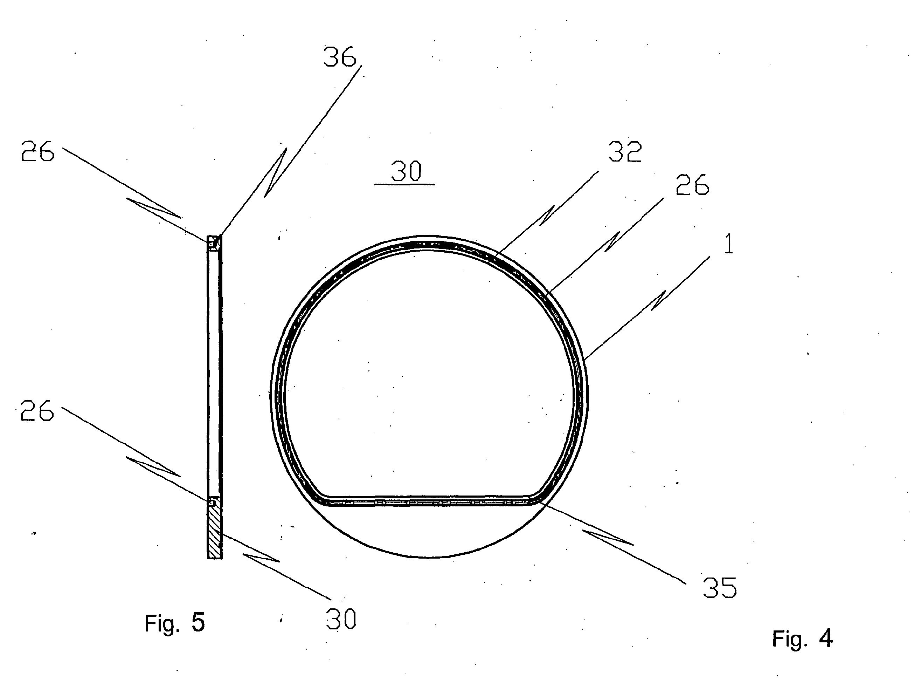

Fig. 4 shows a front view of the opening in an exemplifying embodiment of the vertical bearing metal front plate **30** of the cylindrical chamber **1** of the autoclave. The Fig. shows the circular contour of the cylindrical chamber **1** of the autoclave, in front of which the door opening **32** is formed. The door groove **26** for inserting a sealing is formed around the door opening **32**. The door opening **32** has a circular shape in its upper part and a raised edge **35** in its lower part. The door groove **26** follows the shape of the door opening **32** in a distance, wherein a tangent to the circular part of the door groove **26** at the point of intersection with the linear part in the lower part of the vertical bearing metal front plate **30** of the cylindrical chamber **1** of the autoclave forms an obtuse angle with said linear part, so that the sealing in the door groove **26** is not subjected to such a bending as in case of a right angle or even a sharp angle.

Fig. 5 shows a cross sectional view of the front plate of the chamber of the autoclave from Fig. 4. A door groove **26** for inserting a sealing is milled in the front plate **30**. The walls of said door groove **26** converge slightly from the bottom of the groove so that they may firmly fix the sealing (not shown) inside the door groove **26**. A vacuum suction piping **36** and a compressed air inlet piping (not shown) lead into the door groove **26**.

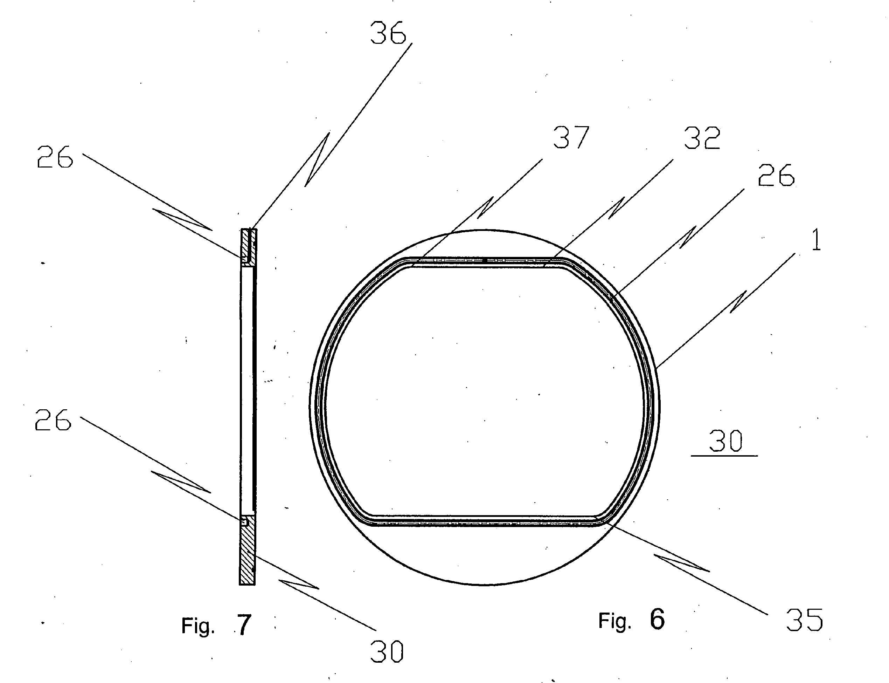

Fig. 6 shows a front view of the opening in another exemplifying embodiment of the vertical bearing metal front plate **30** of the cylindrical chamber **1** of the autoclave. The Fig. shows the circular contour of the cylindrical chamber **1** of the autoclave, in front of which the door opening **32** is formed. The door groove **26** for inserting a sealing is formed around the door opening **32**. The door opening **32** has a circular shape in its side parts and a raised edge **35** in its lower part and a lowered edge **37** in its upper part. The door groove **26** follows the shape of the door opening **32** in a distance, wherein tangents to the circular part of the door groove **26** at the point of intersection with the linear part, i.e. the raised edge **35** in the lower part of the vertical bearing metal front plate **30** of the cylindrical chamber **1** of the autoclave and the lowered edge **37** in the upper part of the vertical bearing metal front plate **30** of the cylindrical chamber **1** of the autoclave form obtuse angles with said linear parts, so that the sealing in the door groove **26** is not subjected to such a bending as in case of a right angle or even a sharp angle. A vacuum suction piping **36** and a compressed air inlet piping (not shown) lead into the door groove **26**.

In operation subjects which shall be sterilized are put into the autoclave through the inlet opening, the door is closed and a heat carrier, e.g. steam, is introduced into the double casing **2.** As the vertical bearing metal plate **30** leans on the floor and there is no wall in front of it and as there are neither control means, nor distribution means in the plate under the inlet opening, the space under the inlet opening of the cylindrical chamber **1** of the autoclave is easy to clean and there is no danger of contamination distribution towards the outlet door and / or the technical equipment of the autoclave. Therefore there is only an easily cleanable plane of the vertical bearing metal plate **30** under the inlet opening of the cylindrical chamber **1.** The opening in the inlet face of the cylindrical chamber **1** of the autoclave has a raised edge in its lower part for keeping the condensate at the bottom of the chamber in case of an open door, so that in case the condensate is not drained before opening the door, the condensate is kept inside the cylindrical chamber **1** of the autoclave even after the door is opened.

The vertical bearing metal plate **30** which serves also as a sealing collar - called SPF collar in specialised publications - is welded to the chamber **1** of the autoclave and forms an integral face of the chamber **1** of the autoclave. It is a bearing structural element of the chamber **1** of the autoclave and an another advantage of its being welded to the chamber **1** of the autoclave is that thermal and mechanical stress inside the autoclave is not transferred to the joint between the chamber **1** and the sealing collar, which is formed by the vertical bearing metal plate **30**. Therefore the joint is not subject to any stress or any risk of defects.

The tube **9** is connected to the vacuum suction piping **4** and arranged as close as possible to the presumed level of condensate, so that non condensing gases may be effectively sucked from the chamber **1** of the autoclave, but at the same time the condensate cannot be sucked away. Then the non condensing gases may be replaced by steam.

Said invention may be used for autoclaves in the form of a steam sterilizer, fan sterilizer using a mixture of steam and air, or a spray type sterilizer using a mixture of water and air.

The control means of the autoclave are connected to the control system, which is provided with equivalent separate HMI panels for a control at any of the autoclave faces. In case of a one-door autoclave the control system comprises one block, in case of a two-doors autoclave the control system comprises two blocks which may be connected in a master - slave or master - master way of operation. Using said control system the vector direction of use of the autoclave is specified, i.e. it is specified which face is the inlet face and which is the outlet face. If it is specified that one face is inlet as well as outlet face, the autoclave functions as a single door autoclave and the second door is not used. The control system controls the processes inside the autoclave as well.

### Industrial applicability

The autoclave according to the invention may be used for sterilization and decontamination of waste as well as of subjects which may be further reused after the decontamination.

## Claims

1. An autoclave, formed by a cylindrical chamber (1), which is closable at at least one face wall of the cylinder, wherein piping for media inlet and media outlet are connected to the cylindrical chamber (1), **characterised in that** the cylindrical chamber (1) of the autoclave has in its lower part a double casing (2), which covers also the waste piping (18) from the cylindrical chamber (1) of the autoclave, and there is a space between two spaced walls of a double casing (2), wherein a heating medium supply leads into said space for an area heating of the lower part of the cylindrical chamber (1) of the autoclave and of the waste piping (18) from the cylindrical chamber (1) of the autoclave, wherein technological elements of the autoclave and the media inlet and media outlet piping for the chamber of the autoclave are arranged above the cylindrical chamber (1) of the autoclave, and at least one face of the cylindrical chamber (1) is formed by a vertical bearing metal plate (30) which is welded to the chamber (1) of the autoclave forming one unit with it and which is provided with a door opening (32), around which, spaced from the door opening edge, a milled groove (26) is provided for a sealing between said vertical bearing metal plate (30) and the chamber door (33), by means of which the face of the autoclave is closable.

2. The autoclave of claim 1, **characterised in that** the two spaced walls of the double casing (2) are connected to each other at its edges only.

3. The autoclave of claim 1, **characterised in that** walls of the milled groove (26) diverge from each other towards the bottom of the milled groove (26).

4. The autoclave according to claim 1, **characterised in that** the door opening (32) in the inlet face of the cylindrical chamber (1) of the autoclave is formed as a part of a circle having a raised edge (35) in its lower part for keeping the condensate at the bottom of the cylindrical chamber when the door (33) of the chamber at the inlet face of the cylindrical chamber (1) of the autoclave is opened.

5. The autoclave of the claim 4, **characterised in that** the door opening (32) in the inlet face of the cylindrical chamber (1) of the autoclave has a lowered edge (37) in its upper part, the lowered edge (37) being arranged parallel to the raised edge (35) of the door opening (32) at the inlet face of the cylindrical chamber (1) of the autoclave.

6. The autoclave according to any of claims 1 to 5, **characterised in that** control means of the autoclave are connected to the control system to specify the direction of use of the autoclave individually for each process and to control processes taking place inside the autoclave.

7. The autoclave according to any of claims 1 to 6, **characterised in that** the chamber door (33) is attached to the vertical bearing metal plate (30) by means of a mechanism (34) for door sliding, which is attached to the vertical bearing metal plate (30) above the level of the raised edge (35) of the door opening (32).

8. The autoclave according to any of claims 1 to 3, **characterised in that** the media distribution inside the cylindrical chamber (1) of the autoclave is effected by means of media inlet and media outlet piping arranged inside the chamber (1) of the autoclave.
